(19) Europäisches Patentamt
European Patent Office
Office européen des brevets

(11) **EP 1 064 923 A1**

(12) **DEMANDE DE BREVET EUROPEEN**

(43) Date de publication:
**03.01.2001 Bulletin 2001/01**

(51) Int Cl.⁷: **A61K 7/42**, A61K 7/48

(21) Numéro de dépôt: **00401622.6**

(22) Date de dépôt: **08.06.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **02.07.1999 FR 9908571**

(71) Demandeur: **L'OREAL**
**75008 Paris (FR)**

(72) Inventeurs:
• **Martin, Richard**
**37210 Rochecorbon (FR)**

• **Belcour-Castro, Béatrice**
**37520 La Riche (FR)**
• **Galup, Cédric**
**92300 Levallois-Perret (FR)**

(74) Mandataire: **Galup, Cédric Olivier Nicolas**
**L'OREAL**
**Départment Propriété Industrielle**
**6, rue Bertrand Sincholle**
**92585 Clichy Cedex (FR)**

(54) **Compositions pour la coloration artificielle de la peau**

(57)    La présente invention se rapporte à des compositions comprenant au moins un extrait d'au moins un végétal du genre Saxifraga à titre d'agent de coloration de la peau.

L'invention concerne également les applications de ces compositions à la coloration de la peau.

EP 1 064 923 A1

Printed by Jouve, 75001 PARIS (FR)

**Description**

La présente invention se rapporte à de nouvelles compositions comprenant au moins un extrait d'au moins un végétal du genre Saxifraga, pour conférer à la peau une coloration artificielle proche du bronzage naturel et à leurs utilisations dans le domaine cosmétique susmentionné.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui sont susceptibles d'induire une altération de la peau, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Cependant, l'utilisation de la DHA peut présenter certains inconvénients. Ainsi, la DHA présente une fâcheuse tendance, plus ou moins prononcée selon la nature du milieu dans lequel elle est formulée, à se dégrader au cours du temps, cette dégradation se traduisant généralement à terme par un jaunissement non souhaitable des compositions qui la contiennent. Un tel phénomène fait que l'activité de la DHA, et en particulier son aptitude à colorer la peau, peut être diminuée au moment de l'application de ces compositions sur la peau. Ainsi, l'intensité de la coloration obtenue sur la peau peut apparaître comme encore insuffisante.

Un autre inconvénient de la DHA est la lenteur avec laquelle la coloration se développe : il faut en effet compter plusieurs heures (3 à 5 heures en général) pour que soit révélée la coloration. De plus, la coloration produite sur la peau par la DHA est souvent jugée trop jaune par les utilisateurs.

Il existe donc une demande croissante de produits autobronzants agissant rapidement et conférant une coloration plus proche du bronzage naturel.

En vue de répondre à ce besoin, on a proposé d'associer la DHA à divers produits : ainsi, le document WO 95/15742 décrit l'association de la DHA avec des acides aminés. Cependant, l'utilisation de telles associations est très peu pratique dans la mesure où elle nécessite soit une application en deux temps soit des conditionnements séparés complexes. Le document FR-2 726 761 décrit quant à lui l'association de la DHA avec la lawsone et/ou la juglone : là encore cette association est peu satisfaisante, du fait cette fois des risques de sensibilisation qu'elle présente.

Ainsi, on est toujours à la recherche de nouveaux produits et de nouvelles compositions permettant de conférer artificiellement à la peau une coloration proche du bronzage naturel d'une manière simple, efficace, rapide et sans risque.

Or, à la suite d'importantes recherches menées dans le domaine de la coloration artificielle de la peau, la Demanderesse a maintenant découvert que l'utilisation de composés particuliers permet de conférer à la peau une coloration artificielle durable proche du bronzage naturel, et ce, immédiatement après l'application sur la peau.

La présente invention a donc pour objet une nouvelle composition, caractérisée par le fait qu'elle comprend, dans un support physiologiquement acceptable, une quantité suffisante d'au moins un extrait d'au moins un végétal du genre Saxifraga pour colorer la peau.

La présente invention a encore pour objet l'utilisation nouvelle d'au moins un extrait tel que défini ci-dessus dans des, ou pour la fabrication de, compositions destinées à colorer la peau.

La présente invention a également pour objet un procédé de traitement cosmétique de la peau destiné à la colorer, caractérisé en ce qu'il consiste à appliquer sur celle-ci une quantité efficace d'un extrait ou d'une composition cosmétique tels que définis ci-dessus.

Les compositions et les utilisations conformes à l'invention permettent d'obtenir une coloration artificielle proche du bronzage naturel en un laps de temps exceptionnellement court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel et également très résistante à l'eau et au temps (elle peut persister sur la peau plusieurs jours).

D'autres caractéristiques, aspects et avantages de la présente invention apparaîtront à la lecture de la description détaillée qui va suivre.

Les extraits utilisés dans la présente invention sont des extraits d'au moins un végétal du genre Saxifraga tels que décrits par G. BONNIER dans La Grande Flore de Gaston Bonnier EDITIONS BELIN, Nov. 1990. Institut National de la Recherche Agronomique & Delachaux & NIESTLÉ SA. L'invention se rapporte donc aux végétaux du genre Saxifraga tels que décrits par G. BONNIER.

Les végétaux du genre Saxifraga sont connus d'une part pour leur utilisation comme végétaux d'ornement mais également en phytothérapie pour leurs propriétés apéritive, astringente, cholagogue et/ou diurétique.

Toutefois, leur utilisation en tant qu'agent de coloration de la peau tel que défini ci-dessus n'a jamais été décrite.

Les végétaux du genre Saxifraga appartiennent à la famille des Saxifragacées et le genre comporte environ 630 espèces, parmi lesquelles on trouve par exemple et sans limitation *Saxifraga cuneifolia, Saxifraga glaucescens, Saxifraga rotundifolia, Saxifraga granulata, Saxifraga bulbifera, Saxifraga umbrosa, Saxifraga tridactylites.*

L'extrait d'au moins un végétal du genre Saxifraga utilisé selon l'invention peut être un extrait de végétal d'une espèce choisie parmi *Saxifraga cuneifolia, Saxifraga glaucescens, Saxifraga rotundifolia, Saxifraga granulata, Saxifraga bulbifera, Saxifraga umbrosa, Saxifraga tridactylites.*

Préférentiellement selon l'invention l'extrait d'au moins un végétal du genre Saxifraga est un extrait de végétal d'une espèce choisie parmi *Saxifraga glaucescens, Saxifraga rotundifolia, Saxifraga granulata.*

L'extrait d'au moins un végétal du genre Saxifraga utilisé selon l'invention peut être obtenu à partir de matériel végétal issu de plante entière ou de partie de plante comme les feuilles, les tiges, les fleurs, les pétales, les racines ou encore des cellules dédifférenciées.

Par cellules végétales dédifférenciées, on entend toute cellule végétale ne présentant aucun des caractères d'une spécialisation particulière et capable de vivre par elle-même et non en dépendance avec d'autres cellules.

Préférentiellement selon l'invention on utilise de la plante entière, particulièrement de la tige et/ou des feuilles, très particulièrement des feuilles.

L'extrait d'au moins un végétal du genre Saxifraga peut être tout extrait préparé à partir de tout matériel végétal issu d'au moins un végétal du genre Saxifraga cultivé *in vivo* ou issu de culture *in vitro.*

Par culture *in vivo* on entend toute culture de type classique c'est à dire en sol à l'air libre ou en serre ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vitro.*

Préférentiellement selon l'invention on utilise un végétal du genre Saxifraga issu de culture *in vitro.*

Toute méthode d'extraction connue de l'homme du métier peut être utilisée pour préparer l'extrait selon l'invention.

On peut, en particulier, citer les extraits alcooliques, notamment éthanoliques ou encore hydroalcooliques.

Préférentiellement selon l'invention, l'extrait est un extrait aqueux.

On peut également utiliser un extrait préparé par la méthode décrite dans la demande de brevet français n° 95-02379 déposée par la demanderesse. Ainsi, dans une première étape on broie le matériel végétal dans une solution aqueuse à froid et dans une deuxième étape les particules en suspension sont éliminées de la solution aqueuse issue de la première étape. Cette solution aqueuse correspond à l'extrait.

Eventuellement, dans une troisième étape on stérilise la solution aqueuse issue de la deuxième étape.

Cet extrait peut alors être lyophilisé.

La première étape peut être avantageusement remplacée par une opération de congélation simple des tissus végétaux (par exemple à -20°C ou encore à -180°C dans l'azote liquide), suivie d'une extraction aqueuse reprenant les deuxième et troisième étapes ci-dessus décrites.

Le traitement à froid permet de geler les activités enzymatiques, la filtration stérilisante évite la dégradation des actifs par les micro-organismes de l'environnement. Enfin, le véhicule eau est compatible avec les récepteurs ex vivo et facilite les formulations cosmétiques ou pharmaceutiques.

Il est connu que les extraits végétaux contiennent des oxydases responsables, entre autres, de l'oxydation desdits extraits. Or une telle oxydation conduit à une coloration marron foncée des extraits et à une odeur âcre rendant ceux-ci peu compatibles avec leur utilisation en cosmétique. Dans cet ordre d'idée on connaît en particulier une laccase dont le poids moléculaire est supérieur à 100000 daltons.

Ainsi, avantageusement, l'extrait obtenu peut être fractionné par toute méthode de fractionnement connue permettant d'éliminer les oxydases et en particulier la polyphénoloxydase. On peut par exemple filtrer l'extrait de l'invention sur une membrane de dialyse afin d'en éliminer les molécules d'un poids moléculaire supérieur à 100000 daltons. Il est également possible de faire subir à l'extrait un fractionnement par précipitations sélectives.

D'autres méthodes permettent de se prémunir des phénomènes d'oxydation. En particulier, l'extrait peut également être stabilisé. Toute méthode de stabilisation connue peut être utilisée selon l'invention. On peut par exemple stabiliser l'extrait de l'invention par barbotage d'azote pour éliminer l'oxygène dissout ou encore en y ajoutant de la cystéine et/ ou des dérivés soufrés à une concentration finale comprise entre 0,5 g/l et 10 g/l et de préférence entre 1 g/l et 2,5 g/l.

Bien évidemment l'extrait selon l'invention peut être fractionné et stabilisé.

L'extrait peut constituer à lui seul le principe actif des compositions de l'invention.

Un exemple de préparation d'extrait utilisable selon l'invention est donné par ailleurs dans les exemples.

De préférence, l'extrait d'au moins un végétal du genre Saxifraga est présent dans les compositions selon la présente invention dans des proportions suffisantes pour conférer à la peau, après application, une coloration similaire à la coloration obtenue à la suite d'un bronzage naturel. Il est ainsi généralement présent dans des proportions comprises entre 0,05% à 20% en poids par rapport au poids total de la composition, et de préférence comprises entre 0,5% à 10% en poids par rapport au poids total de la composition.

Les compositions visées par la présente invention peuvent bien entendu contenir un ou plusieurs autres agents de coloration de la peau comme par exemple les dérivés mono ou polycarbonylés tels que l'isatine, l'alloxane, la ninhydrine, le glycéraldéhyde, l'aldéhyde mésotartrique, les dérivés de pyrazoline-4,5-dione, ces agents de coloration de la peau pouvant être associés ou non à des colorants directs ou des dérivés indoliques.

Dans un mode de réalisation préféré de l'invention les compositions comprennent en outre de la dihydroxyacétone (DHA).

Ainsi, la présente invention a également pour objet une composition, caractérisée par le fait qu'elle comprend, dans un support physiologiquement acceptable, un extrait d'au moins un végétal du genre Saxifraga tel que défini ci-dessus et un autre agent de coloration de la peau. Préférentiellement selon l'invention, l'agent de coloration de la peau est choisi parmi les dérivés mono ou polycarbonylés tels que l'isatine, l'alloxane, la ninhydrine, le glycéraldéhyde, l'aldéhyde mésotartrique, les dérivés de pyrazoline-4,5-dione, la dihydroxyacétone.

Très préférentiellement, l'agent de coloration de la peau est de la dihydroxyacétone (DHA).

En effet, la dihydroxyacétone et les extraits d'au moins un végétal du genre Saxifraga définis ci-dessus présentent une excellente compatibilité chimique au sein des compositions les contenant ainsi qu'une très bonne complémentarité des colorations qu'ils confèrent à la peau, ce qui permet, en les associant dans des proportions adéquates, de parvenir à une coloration artificielle de la peau remarquablement voisine de la coloration conférée par le bronzage naturel.

L'agent de coloration de la peau est présent dans les compositions selon l'invention dans des proportions permettant à l'association des deux agents de coloration de la peau de conférer à la peau, après application, une coloration la plus proche possible de celle obtenue à la suite d'un bronzage naturel. L'agent de coloration de la peau est ainsi généralement présent en une quantité pondérale comprise entre 0,5% et 10% en poids par rapport au poids total de la composition, et de préférence comprise entre 1% et 7% en poids par rapport au poids total de la composition.

La dihydroxyacétone peut également être appliquée sur la peau sous la forme d'une composition indépendante, séparément de la composition comprenant l'extrait d'au moins un végétal du genre Saxifraga, par exemple avant ou après l'application de cette dernière.

Dans un mode de réalisation préféré du procédé de traitement cosmétique selon l'invention, on applique, dans un premier temps, une composition comprenant de la dihydroxyacétone, puis, dans un deuxième temps, la composition comprenant l'extrait d'au moins un végétal du genre Saxifraga, en vue de nuancer selon la volonté de l'utilisateur la coloration jugée parfois un peu jaune apportée par la dihydroxyacétone seule.

Les compositions selon l'invention peuvent également contenir un ou plusieurs filtres solaires actifs dans l'UVA et/ou l'UVB, hydrophiles ou lipophiles, ou encore des pigments d'oxydes métalliques enrobés ou non.

Les compositions selon l'invention peuvent être des compositions cosmétique ou dermatologiques. Préférentiellement selon l'invention les compositions sont des compositions cosmétiques.

Les compositions conformes à la présente invention peuvent comprendre en outre des adjuvants classiques notamment choisis parmi les corps gras, les solvants organiques, les épaississants ioniques ou non ioniques, les adoucissants, les antioxydants, les agents anti-radicaux libres, les opacifiants, les stabilisants, les émollients, les silicones, les $\alpha$-hydroxyacides, les agents anti-mousse, les agents hydratants, les vitamines, les p0arfums, les conservateurs, les tensioactifs, les charges, les séquestrants, les polymères, les propulseurs, les agents alcalinisants ou acidifiants, les colorants, ou tout autre ingrédient habituellement utilisé dans le domaine, en particulier pour la fabrication de compositions anti-solaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale "Finsolv TN" par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'utilisation d'au moins un extrait d'au moins un végétal du genre Saxifraga conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau (H/E) ou eau-dans-huile (E/H).

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Des exemples concrets, mais nullement limitatifs, illustrant l'invention, vont maintenant être donnés.

Exemple 1 : Préparation d'un extrait de *Saxifraga granulata :*

500 grammes de matériel végétal de *Saxifraga granulata,* partie aérienne correspondant à des tiges et des feuilles, sont plongés dans environ 1 kg d'azote liquide.

Après élimination des tiges, les feuilles sont réduites en poudre par broyage au Turrax à 24000 T/min. durant 1 minute à 4°C (bain de glace).

La poudre obtenue est mélangée à 5 litres de tampon phosphate 0,05 M à pH 8,5.

L'ensemble est agité durant 30 minutes à 4°C, puis centrifugé à 10000 G à 4°C.

Le surnageant est filtré à 0,22 µm (filtration stérilisante).

L'extrait est alors fractionné par ultrafiltration sur membrane de type Sartorius afin d'en éliminer les phénomènes d'oxydation.

L'extrait est ensuite lyophilisé. On obtient ainsi environ 5 grammes d'extrait actif dénommé "extrait lyophilisé".

Exemple 2 : Essai *in vitro* de coloration de la peau :

5 grammes de feuilles fraîches de *Saxifraga granulata* sont coupés à l'aide d'un scalpel puis frottés sur un équivalent de peau artificielle du type vitro-skin commercialisé par la société IMS testing group, de surface 8,41 cm$^2$, préalablement pré-hydraté pendant 24 heures dans une enceinte hermétiquement close renfermant un mélange eau/glycérine (70/30).

Une coloration orange hétérogène est observée.

La couleur de la peau est évaluée avant et après la teinture, dans le système L a b, au moyen d'un Chromamètre de marque Minolta (modèle CR200).

Dans le système L a b, les trois paramètres désignent respectivement l'intensité (L), la nuance (a) et la saturation (b).

Selon ce système, plus la valeur de L est élevée, plus la couleur est claire ou peu intense. Inversement, plus la valeur de L est faible, plus la couleur est foncée ou très intense.

a et b indiquent deux axes de couleurs, a indique l'axe de couleur vert/rouge et b l'axe de couleur bleu/jaune. Des valeurs proches de zéro pour a et b correspondent à des nuances grises.

La coloration ($\Delta$E) peut être calculée en appliquant l'équation suivante :

$$\Delta E = \sqrt{(\Delta L)^2 + (\Delta a)^2 + (\Delta b)^2}$$

dans laquelle :

$$\Delta L = L - L_o \,, \ \Delta a = a - a_o \ et \ \Delta b = b - b_o,$$

L, a, et b représentant respectivement l'intensité, la nuance et la saturation de la peau après coloration, $L_0$, $a_0$ et $b_0$ représentant respectivement l'intensité, la nuance et la saturation de la peau avant coloration.

Plus la valeur de $\Delta E$ est importante, plus la différence de couleur entre la peau non colorée et la peau colorée est importante.

Les valeurs suivantes sont observées :

| $\Delta L$ | $\Delta a$ | $\Delta b$ | $\Delta E$ |
|---|---|---|---|
| -12,90 | -2,50 | 15,70 | 20,47 |

Ces résultats confirment la capacité de *Saxifraga granulata* à colorer la peau dans les tons orangés.

Exemple 3 : Exemples de formulations illustrant l'invention et particulièrement les compositions selon l'invention associant au moins un extrait d'au moins un végétal du genre Saxifraga et de la DHA. Ces compositions ont été obtenues par simple mélange des différents composants.

| Composition A: | |
|---|---|
| Montanov 68®* | 7,5 % |
| Diméthicone | 0,5 % |
| Finsolv TN®® | 15,0 % |
| Propylène glycol | 10,0 % |
| Dihydroxyacétone | 5,0 % |
| Extrait de *Saxifraga granulata* | 5,0 % |
| Conservateurs | qs |
| Eau | qsp 100,0 % |

\* : Mélange cétylstéaryl glucoside/alcool cétylstéarylique vendu par la société Seppic
\*\* : Benzoate d'alcools en C12/C15 vendu par la société Finetex

| Composition B : | |
|---|---|
| Extrait de *Saxifraga granulata* | 7,0 % |
| Ceteareth 30 | 7,0 % |
| Stéarate de glycéryle | 2,0 % |
| Alcool cétylique | 1,5 % |
| Polydiméthylsiloxane | 1,5 % |
| Huile de paraffine | 15,0 % |
| Glycérine codex pure | 20,0 % |
| Eau déminéralisée q.s.p. | 100,0 % |
| Conservateurs | q.s. |

| Composition C : | |
|---|---|
| extrait de *Saxifraga granulata* | 15,0 % |
| Dihydroxyacétone | 1,0 % |
| Ethanol | 30,0 % |
| Eau déminéralisée q.s.p. | 100,0 % |

## Revendications

1. Composition, caractérisée par le fait qu'elle comprend, dans un support physiologiquement acceptable, une quantité suffisante d'au moins un extrait d'au moins un végétal du genre Saxifraga pour colorer la peau.

**2.** Composition selon la revendication 1, caractérisée par le fait que le végétal du genre Saxifraga est d'une espèce choisie parmi *Saxifraga cuneifolia, Saxifraga glaucescens, Saxifraga rotundifolia, Saxifraga granulata, Saxifraga bulbifera, Saxifraga umbrosa, Saxifraga tridactylites.*

**3.** Composition selon la revendication 2, caractérisée par le fait que le végétal du genre Saxifraga est d'une espèce choisie parmi *Saxifraga glaucescens, Saxifraga rotundifolia, Saxifraga granulata.*

**4.** Composition selon l'une quelconque des revendications précédentes, caractérisée par le fait que le matériel végétal est issu de plante entière ou de partie de plante comme les feuilles, les tiges, les fleurs, les pétales, les racines ou encore des cellules dédifférenciées.

**5.** Composition selon la revendication 4, caractérisée par le fait que le matériel végétal est issu des feuilles.

**6.** Composition selon l'une quelconque des revendications précédentes, caractérisée par le fait que le matériel végétal est issu d'au moins un végétal du genre Saxifraga cultivé *in vivo* ou issu de culture *in vitro.*

**7.** Composition selon la revendication 6, caractérisée par le fait que le matériel végétal d'au moins un végétal du genre Saxifraga est cultivé *in vitro.*

**8.** Composition selon l'une quelconque des revendications précédentes, caractérisée par le fait que l'extrait est un extrait aqueux.

**9.** Composition selon l'une quelconque des revendications précédentes, caractérisée par le fait que l'extrait est en une quantité pondérale représentant de 0,05% à 20% du poids total de la composition.

**10.** Composition selon la revendication précédente, caractérisée par le fait que l'extrait est en une quantité pondérale représentant de 0,5% à 10% du poids total de la composition.

**11.** Composition selon l'une quelconque des revendications précédentes, caractérisée par le fait qu'elle comprend en outre un autre agent de coloration de la peau.

**12.** Composition selon la revendication 11, caractérisée par le fait que l'agent de coloration de la peau est choisi parmi les dérivés mono ou polycarbonylés tels que l'isatine, l'alloxane, la ninhydrine, le glycéraldéhyde, l'aldéhyde mésotartrique, les dérivés de pyrazoline-4,5-dione, la dihydroxyacétone.

**13.** Composition selon la revendication 12, caractérisée par le fait que l'agent de coloration de la peau est la dihydroxyacétone.

**14.** Composition selon l'une quelconque des revendications 11 à 13, caractérisée par le fait que l'agent de coloration de la peau est présent dans la composition en une quantité comprise entre 0,5 et 10 %.

**15.** Composition selon la revendication 14, caractérisée par le fait que l'agent de coloration de la peau est présent dans la composition en une quantité comprise entre 1 et 7% en poids, par rapport au poids total de la composition.

**16.** Utilisation d'au moins un extrait d'au moins un végétal du genre Saxifraga dans une composition ou pour la préparation d'une composition, l'extrait ou la composition étant destinés à colorer la peau.

**17.** Procédé de traitement cosmétique pour colorer la peau, caractérisé en ce qu'il consiste à appliquer sur celle-ci une quantité efficace d'un extrait d'au moins un végétal du genre Saxifraga ou d'une composition tels que définis à l'une quelconque des revendications 1 à 15.

**18.** Procédé selon la revendication 17, caractérisé par le fait qu'il comprend les deux étapes suivantes :

- i) on applique sur la peau une composition comprenant de la dihydroxyacétone,
- ii) on applique sur la peau une composition telle que définie à l'une quelconque des revendications 1 à 10.

## RAPPORT DE RECHERCHE EUROPEENNE

**Office européen des brevets**

Numéro de la demande

EP 00 40 1622

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | STN, Serveur de Bases de Données, Karlsruhe, DE, Fichier Biosis, AN=1997:170317 XP002134227 * résumé * | 1-18 | A61K7/42 A61K7/48 |
| A | STN, Serveur de Bases de Données, Karlsruhe, DE, Fichier CAPLUS, AN=1971:91102 XP002134228 * résumé * | 1-18 | |
| A | FR 2 409 751 A (THOREL) 22 juin 1979 (1979-06-22) * le document en entier * | 1-18 | |
| A | PATENT ABSTRACTS OF JAPAN vol. 1999, no. 09 (C, 30 juillet 1999 (1999-07-30) & JP 11 092347 A (KAO CORP), 6 avril 1999 (1999-04-06) * abrégé * | 1-18 | |
| A | PATENT ABSTRACTS OF JAPAN vol. 1995, no. 06 (C, 31 juillet 1995 (1995-07-31) & JP 07 069850 A (KAO CORP), 14 mars 1995 (1995-03-14) * abrégé * | 1-18 | DOMAINES TECHNIQUES RECHERCHES (Int.Cl.7) A61K |

Le présent rapport a été établi pour toutes les revendications

| Lieu de la recherche | Date d'achèvement de la recherche | Examinateur |
|---|---|---|
| LA HAYE | 27 octobre 2000 | Fischer, J.P. |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 00 40 1622

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-10-2000

| Document brevet cité<br>au rapport de recherche | Date de<br>publication | Membre(s) de la<br>famille de brevet(s) | Date de<br>publication |
|---|---|---|---|
| FR 2409751 A | 22-06-1979 | AUCUN | |
| JP 11092347 A | 06-04-1999 | AUCUN | |
| JP 07069850 A | 14-03-1995 | AUCUN | |

EPO FORM P0460

Pour tout renseignement concernant cette annexe : voir Journal Officiel de l'Office européen des brevets, No.12/82